# EUROPEAN PATENT APPLICATION

(11) **EP 2 309 268 A1**
(43) Date of publication of application: **13.04.2011**
(21) Application number: 10176153.4
(22) Date of filing: 10.09.2010
(51) Int. Cl.: G01N 33/545

(54) **Method for immunoassay using latex particles**

(30) Priority: 11.09.2009 JP 2009210605
(71) Applicant: Fujifilm Corporation, Minato-ku Tokyo 106-0031 (JP)
(72) Inventor: Kasagi, Noriyuki, Kanagawa 258-8577 (JP); Kuwabara, Tomoko, Kanagawa 258-8577 (JP); Watanabe, Yuya, Kanagawa 258-8577 (JP)
(74) Representative: HOFFMANN EITLE

(57) **Abstract**

An object of the present invention is to provide latex particles with high production reproducibility, by which a sufficiently high value of signal ΔS (= value of signal - value of noise) can be realized in immunodiagnosis. The present invention provides a method for assay which comprises steps of (1) causing a substance to be detected to come into contact with or compete with binding substance-labeled fluorescent latex particles which are obtained by causing a binding substance to bind to fluorescent latex particles, and (2) measuring fluorescence from the binding substance-labeled fluorescent latex particles, wherein: (a) the fluorescent latex particles are obtained by adding a fluorescent substance to latex particles **characterized in that** the amount of COOH groups existing on the surfaces of the latex particles is from 40 µeq/g to 300 µeq/g, or (b) the fluorescent latex particles contain latex particles and a fluorescent substance, which are **characterized in that** the amount of COOH groups existing on the surfaces of the fluorescent latex particles is from 40 µeq/g to 300 µeq/g.

## Description

### Technical Field

The present invention relates to a method for immunoassay using latex particles.

### Background Art

Conventionally, fluorescence detection has been broadly used as a simple and highly sensitive measurement method for biomeasurement and the like. Fluorescence detection is a method that involves: irradiating a sample which is considered to contain a substance to be detected, which emits fluorescence when excited by light with a specific wavelength, with excitation light having such specific wavelength; and detecting fluorescence at such time, so as to confirm the presence of the substance to be detected. Also, the following is widely performed: when a substance to be detected is not a fluorescent material, a fluorescent-dye-labeled substance specifically binding to a substance to be detected is caused to come into contact with a sample; and then fluorescence is detected in the same manner as that described above, so as to confirm that binding has taken place; that is, the presence of the substance to be detected.

Also, regarding such fluorescence detection, a method that uses the effects of electric field enhancement based on plasmon resonance is proposed in JP Patent Publication (Kokai) No. 10-307141 (1998) A and the like in order to improve sensitivity. This method involves, for the generation of plasmon resonance, providing a sensor chip provided with a metal layer within a given region on a transparent support, causing excitation light to enter at a given angle greater than the total angle of reflection from the side of a face opposite to the face (of the support) on which the metal layer is formed with respect to the boundary between the support and a metal membrane; generating a surface plasmon on the metal layer by irradiation with the excitation light; and thus exciting a fluorescent substance using the effects of electric field enhancement.

### Summary of the Invention

The aforementioned conventional immunodiagnosis is still problematic in that a sufficient signal value (ΔS (= value of signal - value of noise)) cannot be obtained. An object to be achieved by the present invention is to resolve the problems of the conventional art described above. Specifically, an object to be achieved by the present invention is to provide a method for immunoassay by which a sufficiently high value of signal ΔS (= value of signal - value of noise) can be realized in immunodiagnosis using latex particles with high production reproducibility, and a method for producing such latex particles.

As a result of intensive studies to achieve the above object, the present inventors have discovered that: latex particles with high production reproducibility can be provided by adjusting the amount of COOH groups existing on the surfaces of latex particles to 40-300 µeq/g; and an immunoassay can be performed, by which a sufficiently high value of signal ΔS (= value of signal - value of noise) can be realized with the use of such latex particles. The present invention has been completed based on these findings.

The present invention provides a method for assay which comprises steps of (1) causing a substance to be detected to come into contact with or compete with binding substance-labeled fluorescent latex particles which are obtained by causing a binding substance to bind to fluorescent latex particles, and (2) measuring fluorescence from the binding substance-labeled fluorescent latex particles, wherein:
(a) the fluorescent latex particles are obtained by adding a fluorescent substance to latex particles **characterized in that** the amount of COOH groups existing on the surfaces of the latex particles is from 40 µeq/g to 300 µeq/g, or
(b) the fluorescent latex particles contain latex particles and a fluorescent substance, which are **characterized in that** the amount of COOH groups existing on the surfaces of the fluorescent latex particles is from 40 µeq/g to 300 µeq/g.

Preferably, the latex is obtained by soap-free polymerization.

Preferably, the latex is a copolymer containing styrene as a monomer.

Preferably, the latex is a copolymer of styrene, and acrylic acid or methacrylic acid.

Preferably, the average particle size of latex particles and/or fluorescent latex particles is from 150 nm to 330 nm.

Preferably, the average particle size of latex particles and/or fluorescent latex particles is from 190 nm to 270 nm.

Preferably, the binding substance is an antibody against a substance to be detected. Preferably, in step (2), fluorescence is measured by surface plasmon fluorescence assay or epifluorescence assay.

The present invention further provides a method for producing latex particles in which the amount of COOH groups existing on the surfaces of latex particles is from 40 µeq/g to 300 µeq/g, which comprises performing polymerization by adding a polymerization initiator dropwise to an aqueous suspension containing styrene and acrylic acid or methacrylic acid wherein a concentration of the styrene is 1.4M or less.

Preferably, the aqueous suspension is raised to 75°C to 95°C before addition of the polymerization initiator.

Preferably, the polymerization initiator is potassium persulfate.

Preferably, polymerization is performed in the presence of a crosslinking agent. According to the present invention, latex particles can be provided that can exert sufficient performance in an immunoassay by adjusting the amount of COOH groups on the surfaces of latex particles to 40 µeq/g-300 µeq/g, In an immunoassay using the fluorescent latex particles of the present invention, the background due to nonspecific adsorption is extremely low, so that high sensitivity evaluation can be performed for the value of signal, Also, the latex particles of the present invention are advantageous in that their production reproducibility is high.

### Brief Description of the Drawings

Fig. 1 shows the relationship ([M] = 2.9 M) between the amount of carboxylic acid introduced and the particle size.
Fig. 2 shows the relationship between the amount of carboxylic acid introduced and the particle size.
Fig. 3 shows the results of SPF immunoassay using fluorescent latex particles.
Fig. 4 shows the results of measuring particle size distribution before and after introduction of a fluorescent dye.
Fig. 5 shows the results of measuring Zeta potentials before and after introduction of a fluorescent dye.

Preferred Embodiments of the Invention

The present invention will be further described in detail as follows.

### (1) Latex particles used in the present invention

Latex particles to be used in the present invention are **characterized in that** the amount of COOH groups on the surfaces is from 40 µeq/g to 300 µeq/g. The amount of COOH groups on the surfaces of latex particles can be measured by measuring electric conductivity. Specifically, the amount of COOH groups on the latex surface can be determined by performing conductometric titration according to a method described in Journal of Colloid and Interface Science 49(3), pp 425-432, 1974 using a pH meter (e.g., a pH meter D-54 (HORIBA, Ltd.)). Specifically, determination is performed as described below. A latex sample, the weight of which has been precisely measured, is diluted with deionized water. A set of both a pH meter and an electrode of a Thomas-Serfass electric conductivity apparatus is placed in a diluted latex dispersion liquid. Latex is stirred and then the pH of the latex sample is adjusted with a dilute NaOH solution to pH 11.5 ± 0.2. The sample is increased by 0.50 ml at a time with 0.421 N H₂SO₄ for titration. Electric conductivity and pH are analyzed as functions of acid consumption until the pH reaches about 2.4.

In addition, an amount of COOH groups ranging from 40 µeq/g to 300 µeq/g is synonymous with an amount of COOH groups ranging from 40 µmol/g to 300 µmol/g.

Specific examples of the latex material include polystyrene, a styrene-acrylic acid copolymer, a styrene-methacrylic acid copolymer, a styrene-glycidyl(meth)acrylate copolymer, a styrene-styrene sulfonate copolymer, a methacrylic acid polymer, an acrylic acid polymer, an acrylonitrile-butadiene-styrene copolymer, a vinyl chloride-acrylic acid ester copolymer, and polyvinyl acetate acrylate. The latex is preferably a copolymer containing at least styrene as a monomer and is particularly preferably a copolymer of styrene, and acrylic acid or methacrylic acid.

Methods for producing latex are not particularly limited, and latex can be produced by any polymerization method. However, since antibody immobilization becomes difficult when a surfactant is present during the labeling of an antibody, soap-free polymerization is preferred for latex production.

The average particle size of latex particles differs depending on particle material, the concentration range for determination of a substance to be detected, measurement instruments, and the like. The average particle size is preferably from 150 nm to 330 nm, and further preferably from 190 nm to 270 nm.

Regarding the relationship between the average particle size of latex particles and the amount of surface COOH groups, latex particle size generally depends on the amount of surface COOH groups. The lower the amount of surface COOH groups, the higher the average particle size. This is because the lower the amount of carboxylic acid, the lower the charge repulsion among latex particles. Moreover, this is particularly because, in a soap-free polymerization system, it becomes difficult for latex to stably exist in water. Hence, for synthesis of latex particles having a particle size within a preferred range and having an amount of surface COOH groups within a preferred range in the present invention, polymerization is preferably performed via a process for production of latex particles as described below or a method based thereon.

### (2) Process for production of latex particles

Latex particles having an amount of COOH groups (existing on their surfaces) ranging from 40 µeq/g to 300 µeq/g, which are used in the present invention, contain, according to a particularly preferred embodiment, styrene and acrylic acid or methacrylic acid. Furthermore, such latex particles can be produced by adding a polymerization initiator dropwise to an aqueous suspension (in which the concentration of styrene is 1.4 M or less) so as to perform polymerization. The use of an aqueous suspension in which the concentration of styrene is higher than 1.4 M is not preferred, since latex particles generated in the polymerization system bind to each other, resulting in an increased average particle size for the generated latex.

Also, the temperature of an aqueous suspension is preferably raised to 75°C-95°C before the addition of a polymerization initiator. Through such temperature increase, the initiator is decomposed simultaneously with the addition thereof and then radicals in the polymerization system are generated at once, so that monomer consumption can be initiated. Such regulation of temperature is preferred, since it has effects of equalizing latex particle size.

A polymerization initiator to be used in the present invention is not particularly limited, as long as polymerization can be performed therewith, and known polymerization initiators can be used. For example, polymerization can be performed using potassium persulfate, 2,2'-azobisisobutyronitrile, 2,2'-azobis(2,4-dimethyl)valeronitrile, 2,2'-azobis4-methoxy-2,4-dimethylvaleronitrile, benzoyl peroxide, 2,4-dichloroperoxide, isopropyl peroxycarbonate, cumene hydroperoxide, lauroyl peroxide, or the like. Particularly preferably, polymerization can be performed using potassium persulfate. The amount of a polymerization initiator to be used herein is preferably about 0.1% to 5% by weight of a monomer composition.

Also, polymerization can be performed in the presence of a crosslinking agent. Examples of a crosslinking agent that can be used herein include, but are not limited to, divinylbenzene and 1,4 butadiene.

### (3) Fluorescent latex particles

Latex particles to be used in the present invention are preferably fluorescent substances. When latex itself obtained by polymerization as described in (2) above is fluorescent, the particles of such latex can be directly used as fluorescent latex particles. When latex obtained by polymerization is non-fluorescent, fluorescent latex particles can be produced by adding a fluorescent substance (e.g., fluorescent dye) to the latex. Specifically, fluorescent latex particles can be produced by adding a fluorescent dye to a solution of latex particles containing water and a water soluble organic solvent and then stirring the mixture, for example. The concentration of latex in a solution of latex particles is preferably from 0.1% by weight to 10% by weight. The solution preferably contains an electrolyte. As an electrolyte, NaCl is preferred, and the concentration of an electrolyte in a solution is preferably from 1 mM to 500 mM. Also, as a water soluble organic solvent contained in a solution of latex particles, tetrahydrofuran (THF), dimethylformiamide (DMF), dimethylacetamide (DMAc), or acetone is preferred. The percentage of a water soluble organic solvent to water is preferably about from 10% to 80%.

The term "fluorescent latex particles containing latex particles and a fluorescent substance" as used herein refers to both: fluorescent latex particles when latex itself obtained by polymerization as described in (2) above is fluorescent; and fluorescent latex particles obtained by addition of a fluorescent substance (e.g., fluorescent dye) to latex when the latex obtained by polymerization is non-fluorescent.

### (4) Binding substance-labeled latex particles

The latex particles of the present invention can be used in fluorescence assay by causing a binding substance to bind to the latex particles. Here, the term "binding substance" used herein may refer to a substance that binds to a sensor part via a substance to be detected or a competitive substance that binds to a sensor part competitively with a substance to be detected. For example, when an antigen is a substance to be detected in fluorescence assay for detection of an antigen-antibody reaction and an assay is performed by a sandwich method, an immobilization layer is composed of a primary antibody (immobilized antibody) specifically binding to the antigen and a binding substance is composed of a second antibody specifically binding to the antigen. Also, when an assay is performed by a competition method, a binding substance may be composed of an antibody specifically binding to an antigen or a competitive antigen binding to an immobilized antibody competitively with an antigen.

When a binding substance is a second antibody specifically binding to an antigen, examples of such antibody specifically binding to an antigen are not particularly limited. For example, antiserum prepared from the serum of an animal immunized with a substance to be detected, an immunoglobulin fraction purified from antiserum, a monoclonal antibody obtained by cell fusion using spleen cells of an animal immunized with a substance to be detected, or fragments thereof [e.g., F(ab')2, Fab, Fab', or Fv] can be used. Such antibody can be prepared by a conventional method. Moreover, such antibodies that can be used herein may be modified antibodies such as chimeric antibodies, commercially available antibodies, and antibodies prepared by a known method from animal serum or culture supernatants. Fragmented antibodies can be used regardless of animal species, subclass, and the like. For example, antibodies that can be used in the present invention are antibodies from mice, rats, goats, rabbits, sheep, or the like. Specific examples thereof include mouse IgG, mouse IgM, rat IgG, rat IgM, rabbit IgG, rabbit IgM, goat IgG, goat IgM, sheep IgG, and sheep IgM. Such antibodies may be either polyclonal or monoclonal. A fragmented antibody is a molecule prepared from a complete antibody having at least one antigen binding site. Specific examples thereof include Fab and F(ab')2. These fragmented antibodies are molecules obtained by treatment with enzymes, or chemical treatment, or gene engineering techniques.

Next, a method for binding an antibody to fluorescent latex is described. A solution containing an antibody is added to an aqueous solution of fluorescent latex particles, and then the mixture is stirred. Subsequently, a WSC (Product No. 01-62-0011, Wako Pure Chemical Industries, Ltd.) aqueous solution is added to the solution and then the resultant is stirred. A glycine aqueous solution is further added to the solution, the solution is stirred, and then fluorescent latex particles are precipitated by centrifugation. Supernatants are removed, a PBS solution is added, and then fluorescent latex particles are re-dispersed using an ultrasonic washing machine. Alter removal of supernatants by further centrifugation, a PBS solution containing 1% BSA is added to cause re-dispersion of fluorescent latex particles, so that a solution of antibody-bound fluorescent latex particles can be obtained.

### (5) Assay modes

The present invention further relates to a method for assay which comprises steps of: (1) causing a substance to be detected to come into contact with or to compete with binding substance-labeled fluorescent latex particles obtained by causing a binding substance to bind to the fluorescent latex particles of the present invention; and (2) measuring the fluorescence of the above binding substance-labeled fluorescent latex particles.

The method for assay according to the present invention should be construed according to the broadest possible conception, including detection of the presence or the absence of a substance to be detected or measurement (that is, determination) of the amount of a substance to be detected. Examples of the assay unlimitedly include generally known immunological measurement methods. Hereafter, a sandwich method and a competition method will be described below as specific embodiments of the assay of the present invention, but the present invention is not limited thereto.

### (Sandwich method)

With the use of a sandwich method, a substance to be detected can be measured by the following procedures, for example, but measurement is not limited thereto. First, a primary antibody and a second antibody having specificity to a substance to be detected (antigen) are prepared in advance. Next, the primary antibody is immobilized on a solid phase, such as a substrate, and then the second antibody is caused to bind to fluorescent latex particles, so that antibody-labeled fluorescent latex particles are prepared. Subsequently, a test sample (or an extract thereof) that can contain a substance to be detected (antigen) and antibody-labeled fluorescent latex particles are brought into contact with each other on a substrate. When a substance to be detected is present in a test sample, an antigen-antibody reaction takes place between the substance to be detected and antibody-labeled fluorescent latex particles and between the substance to be detected and a substrate. Such antigen-antibody reaction can be performed in a manner similar to a general antigen-antibody reaction. As a result, when a substance to be detected is present in a test sample, a primary antibody immobilized on the substrate, the substance to be detected (antigen), and a second antibody binding to fluorescent latex particles form an immune complex. In the sandwich method, after a reaction among a primary antibody immobilized on a substrate, a substance to be detected (antigen), and a second antibody binding to fluorescent latex particles is completed, the second antibody that formed no immune complex described above is removed, followed by washing. The degree of formation of the above immune complex is detected as fluorescence intensity, so that the concentration of the substance to be detected and the like can be measured. In addition, there is a positive correlation between fluorescence intensity and the concentration of a substance to be detected.

### (Competition method)

With the use of a competition method, a substance to be detected can be measured by the following procedures, for example, but such measurement is not particularly limited thereto. The competition method is known as a technique for detection of an antigen that is a low molecular weight compound that cannot be assayed by the sandwich method.

First, a primary antibody having specificity to a substance to be detected (antigen) is prepared in advance. Then the primary antibody is caused to bind to fluorescent latex particles. A substance (to be detected) itself having affinity for the primary antibody or a compound having a site analogous to a substance to be detected and an epitope for the primary antibody which is similar to the substance to be detected is immobilized on a solid phase, such as a substrate. Then a test sample (or an extract thereof) that can contain the substance to be detected (antigen) and the primary antibody-bound fluorescent latex particles are brought into contact with the substrate. When no substance to be detected is present in the test sample, an antigen-antibody reaction takes place on the substrate between the primary antibody and the substance (to be detected) itself having affinity for the primary antibody or a compound having an epitope for the primary antibody which is similar to the substance to be detected. On the other hand, when a substance to be detected is present, the substance to be detected (antigen) binds to the primary antibody. Hence, the subsequent antigen-antibody reaction (on the substrate) with the substance (to be detected) itself having affinity for the primary antibody or a compound having a site analogous to the substance to be detected and having an epitope for the primary antibody which is similar to the substance to be detected is inhibited. Thus, binding resulting from such antigen-antibody reaction does not take place. After the reaction between an immobilized material having affinity for the primary antibody and the primary antibody binding to fluorescent latex particles is completed, fluorescent latex particles that have formed no immune complex which was described above are removed. Subsequently, the degree of formation of the above immune complex is detected as fluorescence intensity, so that the concentration of the substance to be detected and the like can be measured.

Alternatively, according to another embodiment, when an assay is performed by a competition method, "a substance to be detected itself, or a compound having a site analogous to a substance to be detected and having an epitope for a primary antibody which is similar to the substance to be detected" is caused to bind to fluorescent latex particles. The primary antibody having specificity to the substance to be detected (antigen) may be immobilized on a solid phase, such as a substrate. Also in this case, the concentration of the substance to be detected and the like can be measured by performing an antigen-antibody reaction in the same way as mentioned above.

### (6) Method for detection of fluorescence

A method for detection of fluorescence in the present invention is not particularly limited. For example, fluorescence intensity can be detected using an instrument capable of detecting fluorescence intensity. A specific example is a microplate reader or a biosensor for surface plasmon fluorescence (SPF) detection using surface plasmon excitation, Detection of fluorescence intensity is generally completed within a given period of time after an antigen-antibody reaction, such as within several minutes to several hours. Through detection of the degree of formation of the above immune complex via fluorescence intensity, the concentration of a substance to be detected can be determined based on the relationship between fluorescence intensity and the concentration of the substance to be detected. The type of measurement of fluorescence may be plate reader measurement or flow measurement. In addition, surface plasmon fluorescence (SPF) detection using surface plasmon excitation can be performed via measurement with sensitivity which is higher than that in the case of a fluorescence detection method using epi-illumination excitation (hereafter, referred to as an "epi-fluorescence method").

A sensor that can be used as the above surface plasmon fluorescence (SPF) biosensor, for example, as disclosed in JP Patent Publication (Kokai) No. 2008-249361A, comprises an optical waveguide formed from a material that excitation light with a given wavelength can penetrate, a metal membrane formed on a surface of the optical waveguide, a light source for generation of an optical beam, an optical system for causing the optical beam to enter the optical waveguide at an angle of incidence by which surface plasmon is generated toward the boundary between the optical waveguide and the metal membrane, and a fluorescence detection means for detecting fluorescence generated as a result of excitation by the evanescent wave enhanced by the surface plasmon.

The surface plasmon fluorescence (SPF) detection system using fluorescent particles of the present invention is different from a latex agglutination method. In the latex agglutination method, antibody sensitized latex in a latex reagent and an antigen in a specimen bind to each other to undergo agglutination as a result of an antibody reaction. The resulting agglutinates increase with time and then the agglutinates are irradiated with far-red light. A manner of quantifying the concentration of the antigen based on the thus obtained change in absorbance per unit time is a latex agglutination method. Agglutinates resulting from an antigen-antibody reaction as a result of immunonephelometry are very small, so that the optical detection of the degree of agglutination within a low-concentration range in cases in which the amount of the antigen is low is difficult Hence, conventionally in a latex agglutination method by which relatively large latex particles (of the µm size) are sensitized with an antibody, an antigen-antibody reaction appears in the form of apparent latex agglutination. Therefore, even when the amount of an antigen within a low-concentration range is low, large agglutinates appear and even slight changes in agglutinates can be optically captured. Examples of such method include a turbidimetric method by which the formation of agglutinates is detected as an increase in turbidity, a method by which the formation of agglutinates is detected as a change in particle size distribution or average particle size, and an integrating sphere turbidity method that comprises measuring a change in forward-scattered light due to the formation of agglutinates using integrating sphere and then comparing the ratio thereof with the intensity of transmitted light. Examples of the above measurement methods include a rate assay that involves obtaining at least two measurement values at different time points, and then finding the degree of agglutination based on the difference (an increase) occurring between these time points (that is, rate of increase), and, an end point assay that involves generally obtaining one measured value at a time point thought to be an end point of the reaction and then finding the degree of agglutination based on the measured value. Examples of an automatic apparatus for clinical laboratory tests, which is appropriate for immunological latex agglutination reaction measurement, include commercially available automatic analyzers such as the Hitachi 7070, 7150, 7170, LPIA-A700, and S500.

The present invention will be further specifically described by the following Examples, but the present invention is not limited to the Examples.

### Examples

Soap-free polymerization is preferred for particle production, since antibody immobilization is difficult when a surfactant is present upon antibody labeling. However, it is difficult to perform soap-free polymerization, when the particle size is 200 nm or less. Also, with a particle size of 300 nm or more, evaluation was impossible because of agglutination after antibody labeling. Therefore, an experiment was conducted with the particle size narrowed down to 200 nm to 250 nm. The results are shown in the following Examples.

In the following Examples, the particle size was measured using the ZETA SIZER (Sysmex) Nano series. Samples were prepared to have a concentration of 0.01 % and then measured using 10 mM Phosphate pH 7.0 (800 µL). The parameters are as follows. Polystyren latex: RI: 1.590; Absorption: 0.01; solvent: water; temperature: 25°C; viscosity, 0.8872 cP; RI: 1.330 Also, the amount of carboxylic acid in latex was determined by performing conductometric titration according to the method described in the Journal of Colloid and Interface Science 49 (3), pp 425-432, 974 using a pH meter D-54 (HORIBA, Ltd.).

### Example 1: Production of latex particles

The results of examining soap-free polymerization using styrene, acrylic acid, and potassium persulfate (KPS) are as described below.

### (1) Examination of the amount of an initiator (potassium persulfate: KPS)

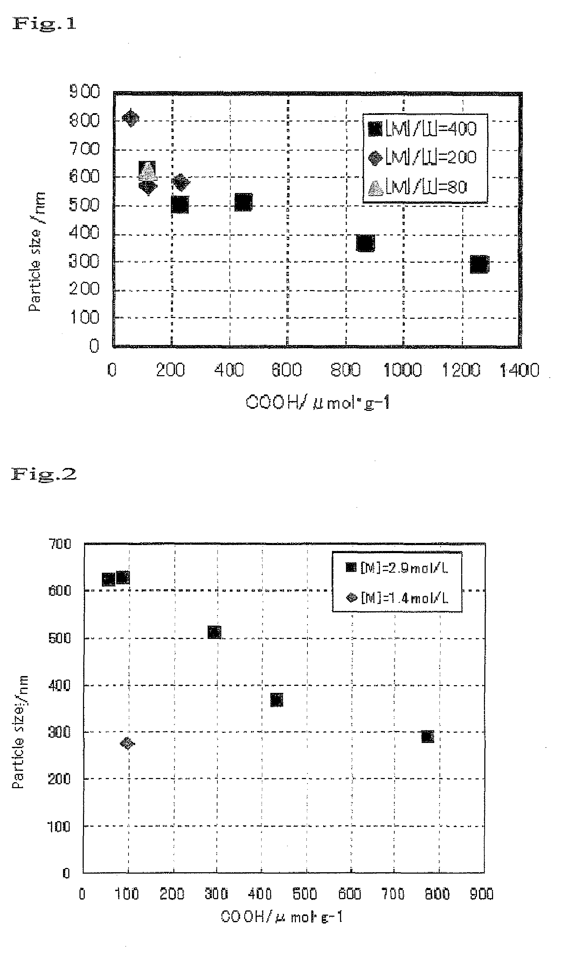

Fig. 1 shows the relationship between the amount of carboxylic acid on particle surfaces and the particle size ([M] = 2.9 M). At a monomer concentration of 2.9 mol.L-1, particle size was determined depending on the amount of carboxylic acid on particle surfaces, independent of the amount of the initiator (the ratio of [M]/[I] was introduced).

### (2) Synthesis example of latex particles (diameter: 511 nm; and COOH group at 146 µmol/g)

Styrene (Wako Pure Chemical Industries, Ltd.) (30 g) (288 mmol) and 1 g (56 mol) of acrylic acid (Wako Pure Chemical Industries, Ltd.) were suspended and dissolved in 100 mL of milli-Q water. An aqueous solution prepared by dissolving 0.2 g of KPS (Wako Pure Chemical Industries, Ltd.) in 10 mL at 85°C was added thereto, followed by 6 hours of stirring at 65°C and 250 rpm. Subsequently, 3 hours of centrifugation was performed 3 times at 8000 rpm and then the resultant was finally re-dispersed in milli-Q water.

### (3) Examination of the concentration of a monomer (styrene) to be introduced

Fig. 2 shows the results of examining the concentration of a monomer (styrene) to be introduced. When the concentration for introduction was lowered, the amount of carboxylic acid introduced was low and the production of particles having a particle size of 275 nm became possible.

### (4) Production of latex particles

A method for producing latex particles having the physical properties listed in Table 1 below is described as follows,

**Table 1:**

| Sample No. | Particle size (nm) | Amount of COOH group (µeq./g) |
|---|---|---|
| (1) | 203 nm | 56 |
| (2) | 199 nm | 265 |
| (3) | 214 nm | 420 |
| (4) | 264nm | 417 |
| (5) | 254 nm | 84 |

Production of sample No. (1) Styrene (Wako Pure Chemical Industries, Ltd.) (30 g) (288 mmol) and 0.5 g (7 mmol) of acrylic acid (Wako Pure Chemical Industries, Ltd.) were suspended in 660 mL of milli-Q water, the temperature was raised to 85°C, and then an aqueous solution prepared by dissolving 1 g of KPS (Wako Pure Chemical Industries, Ltd.) in 10 mL was added thereto, followed by 6 hours of stirring at 85°C and 250 rpm. Subsequently, 3 hours of centrifugation was performed 3 times at 10,000 rpm and then the resultant was finally re-dispersed in milli-Q water.

### Production of sample No. (2)

Styrene (Wako Pure Chemical Industries, Ltd.) (30 g) (288 mmol) and 3 g (42 mmol) of acrylic acid (Wako Pure Chemical Industries, Ltd.) were suspended in 440 mL of milli-Q water, the temperature was raised to 85°C, and then an aqueous solution prepared by dissolving 1 g of KPS (Wako Pure Chemical Industries, Ltd.) in 10 mL was added thereto, followed by 6 hours of stirring at 85°C and 250 rpm. Subsequently, 3 hours of centrifugation was performed 3 times at 10,000 rpm and then the resultant was finally re-dispersed in milli-Q water.

### Production of sample No. (3)

Styrene (Wako Pure Chemical Industries, Ltd.) (30 g) (288 mmol) and 6 g (84 mmol) of acrylic acid (Wako Pure Chemical Industries, Ltd.) were suspended in 330 mL of milli-Q water, the temperature was raised to 85°C, and then an aqueous solution prepared by dissolving 1 g of KPS (Wako Pure Chemical Industries, Ltd.) in 10 mL was added thereto, followed by 6 hours of stirring at 85°C and 250 rpm. Subsequently, 3 hours of centrifugation was performed 3 times at 10,000 rpm and then the resultant was finally re-dispersed in milli-Q water.

### Production of sample No. (4)

Styrene (Wako Pure Chemical Industries, Ltd.) (30 g) (288 mmol) and 6 g (83 mmol) of acrylic acid (Wako Pure Chemical Industries, Ltd.) were suspended in 220 mL of milli-Q water, the temperature was raised to 85°C, and then an aqueous solution prepared by dissolving 1 g of KPS (Wako Pure Chemical Industries, Ltd.) in 10 mL was added thereto, followed by 6 hours of stirring at 85°C and 250 rpm. Subsequently, 3 hours of centrifugation was performed 3 times at 10,000 rpm and then the resultant was finally re-dispersed in milli-Q water.

### Production of sample No. (5)

Styrene (Wako Pure Chemical Industries, Ltd.) (30 g) (288 mmol) and 1 g (14 mmol) of acrylic acid (Wako Pure Chemical Industries, Ltd.) were suspended in 660 mL of milli-Q water, the temperature was raised to 85°C, and then an aqueous solution prepared by dissolving 1 g of KPS (Wako Pure Chemical Industries, Ltd.) in 10 mL was added thereto, followed by 6 hours of stirring at 85°C and 250 rpm. Subsequently, 3 hours of centrifugation was performed 3 times at 10,000 rpm and then the resultant was finally re-dispersed in milli-Q water.

### Example 2: Production of fluorescent latex particles

Latex particles (sample Nos. (1)-(5) as listed in Table 1) (13.4% solid, 745 µL) were dissolved in 9.255 mL of 500 mM NaCl aqueous solution, and then 10 mL of dimethylformamide (DMF) was added, followed by 30 minutes of stirring at room temperature with shielding from light. Subsequently, 0.5 mg (50 µ DMF solution) of a fluorescent dye BODIPY650/665 (Invitrogen) was added, and then the resultant was stirred at room temperature for 30 minutes. Purification was performed by repeating centrifugation (15,000 rpm, 60 minutes, 4°C) and re-dispersion into 1×PBS 3 times. Finally, 5 mL of milli-Q water was added, so as to adjust the solid content concentration to 2% solid.

### Example 3: Production of fluorescent latex particles to which antibody was bound

250 µL of 50 mM MES buffer (pH 6.0) solution containing 2 mg/mL anti-hCG monoclonal antibody Anti-alpha subunit 6601 SPR-5 (IgG2a (Medix)) was added to 250 µL of an aqueous solution of 2% (solid content concentration) fluorescent latex particles prepared in Example 2, followed by 15 minutes of stirring at room temperature. Subsequently, 5 µL of a 10 mg/mL WSC (Product No. 01-62-0011, Wako Pure Chemical Industries, Ltd.) aqueous solution was added and the solution was stirred at room temperature for 2 hours. 2 mol/L glycine aqueous solution (25 µL) was added thereto and then the solution was stirred for 30 minutes. Fluorescent latex particles were then precipitated by centrifugation (15,000 rpm, 4°C, 15 minutes). Supernatants were removed, 500 µL of a PBS solution (pH 7.4) was added, and then fluorescent latex particles were re-dispersed using an ultrasonic washing machine. Centrifugation (15,000 rpm., 4°C, 15 minutes) was further performed and then supernatants were removed. PBS (pH 7.4) solution (500 µL) containing 1% BSA was then added, and then fluorescent latex particles were re-dispersed, so that a 1% (w/v) solution of anti-hCG antibody-bound fluorescent latex particles was obtained.

### Example 4: Examination of SPF immunoassay in the flow system (assay system using hCG antigen)

Regarding antibodies of sandwich pair, anti-hCG 5008 SP-5 JgG1) (Medix) was used on the solid phase and anti-alpha subunit 6601 SPR-5 (IgG2a (Medix)) was used on the fluorescent particles.

An antibody solution was spotted onto a gold film using a chip comprising polymethylmethacrylate as a substrate on which a gold film had been deposited, so that the antibody was immobilized. Subsequently, immunoassay was performed according to the following procedures by using antibody-labeled fluorescent particles prepared in Example 3.

Before attachment of an upper wall onto the top of a fluid path of the sensor chip, 100 µL of 150 mM sodium chloride solution of anti-hCG monoclonal antibody Anti-hCG 5008 SP-5 (IgG1) (Medix) adjusted to 10 µg/mL was added to a measurement area of the sensor chip. The sensor chip was left to stand at room temperature for 1 hour. The antibody solution was removed and then the sensor chip was washed with a previously prepared washing buffer (PBS (pH 7.4) containing 0.05% (w/v) Tween-20) (300 µL/washing, 3 times). After washing, to block the unadsorbed portion of the antibody, 300 µL of PBS (pH 7.4) containing 1% casein was added and then the resultant was left to stand for 1 hour at room temperature. After washing with the above washing buffer, 300 µL of Immunoassay Stabilizer (ABI) was added as a stabilizer to each well, and then the wells were left to stand at room temperature for 30 minutes. The solution was removed and then moisture was completely removed in a dryer. After treatment of binding of the anti-hCG antibody, the fluid path of the sensor chip was sealed using a lid material, and then a fluid path sensor chip was prepared. Ultrasonic deposition or the like can be employed for sealing of the fluid path.

PBS solutions (phosphate buffer) of 1% BSA which contain 0 pM, 0.9 pM, 9 pM, or 90 pM purified hCG antigen were prepared, and were used as sample solutions. The concentration of 0 pM indicates the 1% BSA PBS solution itself.

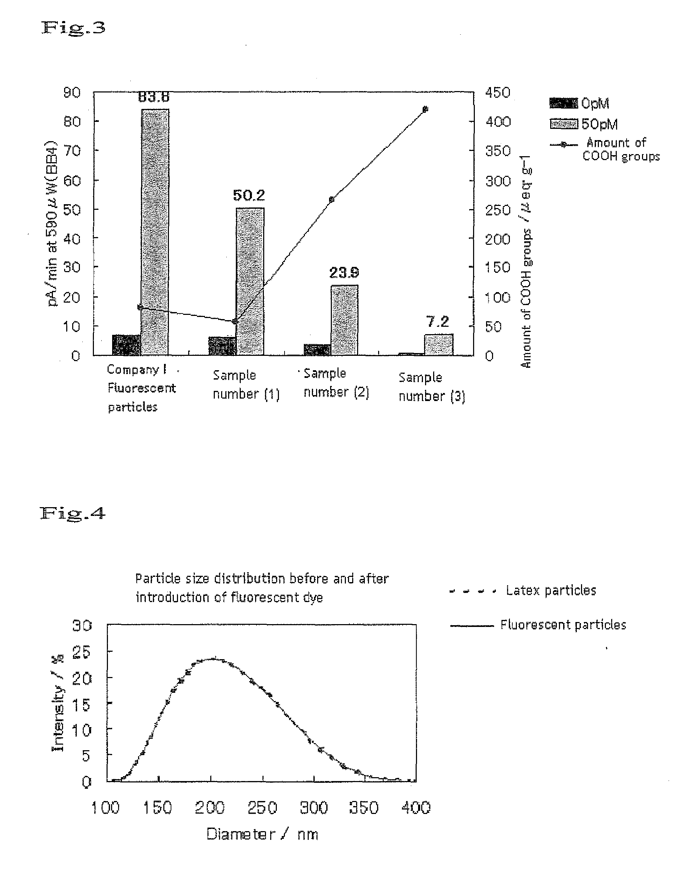

To 500 µL of each sample solution, 5 µL each of the 1% anti-hCG antibody-bound fluorescent label solution prepared by the above procedures was added and mixed, so as to prepare a reaction solution. Fluorescent signals from the measurement area were measured at a plurality of different time points while causing the reaction solution to flow down on the measurement area using a sample cell. Fluorescent signals were measured by irradiating the region in which a complex (antibody-antigen-antibody) was formed on the gold film with an NIR laser from the side at which no antibody was bound so as to generate an evanescent wave. An evanescent wave reached only a region in the vicinity of the gold film and then excited mainly the label contained in the above sandwich complex, so as to emit fluorescence. The fluorescence was detected using a photodiode. At this time, a pump was connected to an air hole of the sample cell and pump suction was performed at a constant flow rate (linear velocity) of 1.4 mm/s. Measurement was performed while sending 300 µL of the reaction solution onto the measurement area. Fig. 3 shows the results of evaluating the density of trapped antigens using a blood plasma system in SPF measurement, in order to evaluate the performance of immunoassay. Specifically, Fig. 3 shows the results of comparing each type of fluorescent particles using ΔS = value of signal - value of noise.

Reaction conditions:
Primary reaction: 10 minutes;
Flow rate: 10 µL/minute;
Flow time: 5 minutes;
Washing time: 5 minutes

### Example 5: Examination of reproducibility and appropriate production method of sample (2) exerting good immunoassay performance in SPF detection method

Styrene (Wako Pure Chemical Industries, Ltd.) (30 g) (288 mmol) and 4 g (56 mmol) of acrylic acid (Wako Pure Chemical Industries, Ltd) were suspended in 480 mL of milli-Q water, the temperature was raised to 85°C, and then an aqueous solution prepared by dissolving 1.16 g of KPS (Wako Pure Chemical Industries, Ltd.) in 10 mL was added thereto, followed by 6 hours of stirring at 85°C and 250 rpm, Subsequently, 3 hours of centrifugation was performed 3 times at 10,000 rpm, and then the resultant was finally re-dispersed in milli-Q water.

The results of examining polymerization reproducibility under conditions of [M]/[I] = 80 M, [St]/[AA]= 5.12, [M] = 1.72 M, 80°C, and 4 hours are shown in Table 2 below.

**Table 2:**

| Sample No. | Particle size/nm (DLS) | COOH/µeq·g-1 (electric conductivity) | Mn(Mw/Mn) (GPC) | Remaining monomer (styrene)/% (¹H-NMR) |
|---|---|---|---|---|
| (6) | 229 | 252 | 20.700(2.98) | <0.1% |
| (7) | 223 | 290 | 20.500(2.89) | <0.1% |
| (8) | 224 | 269 | 23.800(2.86) | <0.1% |

Three samples were produced. A formulation with good reproducibility could be established wherein the particle size is 225±5 nm and the amount of COOH groups is within 270±20 µeq/g.

### Example 6:

Examination of formulation for synthesis of latex particles (particle size of 200-210 nm; around 60-120 µmol/g) Polymerization reproducibility was examined under conditions of [M]/[I]=80, M=0.45mol·L-¹, [St]/[AA]=26.18, 80°C, and 6 hours.

**Table 3:**

| Lot No. | Particle size/nm (DLS) | COOH/µeq·g-¹ (electric conductivity) | Mn (Mw/Mn) (GPC) | Remaining monomer (styrene)/% (¹H-NMR) |
|---|---|---|---|---|
| (9) | 222 | 106 | 17,100 (4.21) | <0.1% |
| (10) | 223 | * | 43,300 (5.37) | 1.3 |
| (11) | 219 | * | 23,100 (4.78) | 2.4 |

| | | | | |
|---|---|---|---|---|
| *Unmeasured | | | | |

Reproducibility of the particle size was satisfactory, but the molecular weight was unstable and monomers remained. It is difficult to achieve polymerization reproducibility in case of latex particles having low amounts of carboxylic acid.

### Example 7: Particle size distribution and surfaces COOH amount before and after introduction of fluorescent dye

Fig. 4 shows the results of examining the particle size distribution before and after introduction of a fluorescent dye (BODIPY650/665: Invitrogen). It was demonstrated that the particle size did not change before and after the introduction of a fluorescent dye.

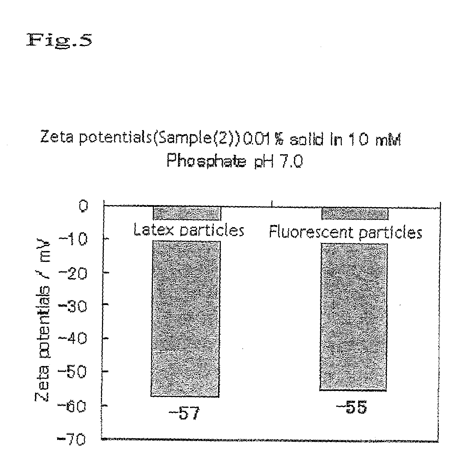

Also, electric conductivity cannot be measured after introduction of a fluorescent dye. The surface COOH amount was evaluated before and after the introduction of a fluorescent dye (BODIPY650/665: Invitrogen) using Zeta potential as an index. Fig. 5 shows the results. The surface COOH amount did not substantially change before and after the introduction of then fluorescent dye.

## Claims

1. A method for assay which comprises steps of (1) causing a substance to be detected to come into contact with or compete with binding substance-labeled fluorescent latex particles which are obtained by causing a binding substance to bind to fluorescent latex particles, and (2) measuring fluorescence from the binding substance-labeled fluorescent latex particles, wherein:
(a) the fluorescent latex particles are obtained by adding a fluorescent substance to latex particles **characterized in that** the amount of COOH groups existing on the surfaces of the latex particles is from 40 µeq/g to 300 µeq/g, or
(b) the fluorescent latex particles contain latex particles and a fluorescent substance, which are **characterized in that** the amount of COOH groups existing on the surfaces of the fluorescent latex particles is from 40 µeq/g to 300 µeq/g.

2. The method for assay according to claim 1, wherein the latex is obtained by soap-free polymerization.

3. The method for assay according to claim 1, wherein the latex is a copolymer containing styrene as a monomer.

4. The method for assay according to claim 1, wherein the latex is a copolymer of styrene, and acrylic acid or methacrylic acid.

5. The method for assay according to claim 1, wherein the average particle size of latex particles and/or fluorescent latex particles is from 150 nm to 330 nm.

6. The method for assay according to claim 1, wherein the average particle size of latex particles and/or fluorescent latex particles is from 190 nm to 270 nm.

7. The method for assay according to claim 1, wherein the binding substance is an antibody against a substance to be detected.

8. The method for assay according to claim 1, wherein in step (2), fluorescence is measured by surface plasmon fluorescence assay or epifluorescence assay.

9. A method for producing latex particles in which the amount of COOH groups existing on the surfaces of latex particles is from 40 µeq/g to 300 µeq/g, which comprises performing polymerization by adding a polymerization initiator dropwise to an aqueous suspension containing styrene and acrylic acid or methacrylic acid wherein a concentration of the styrene is 1.4 M or less.

10. The method according to claim 9, wherein the aqueous suspension is raised to 75°C to 95°C before addition of the polymerization initiator.

11. The method according to claim 9, wherein the polymerization initiator is potassium persulfate.

12. The method according to claim 9, wherein polymerization is performed in the presence of a crosslinking agent.
